# EUROPEAN PATENT SPECIFICATION

(11) **EP 2 765 934 B1**
(45) Date of publication and mention of the grant of the patent: **09.09.2015**
(21) Application number: 12780436.7
(22) Date of filing: 12.10.2012
(51) Int. Cl.: A61B 17/70

(54) **BONE ANCHOR ASSEMBLIES**
KNOCHENANKERANORDNUNGEN
ENSEMBLES ANCRES OSSEUSES

(30) Priority: 14.10.2011 US 201161547274 P
(43) Date of publication of application: 20.08.2014
(73) Proprietor: Medos International Sarl, 2400 Le Locle (CH)
(72) Inventor: CHANDANSON, Thibault, 2400 Le Locle (CH); DEFOSSEZ, Henri, 2400 Le Locle (CH); SPRATT, Frank, 2400 Le Locle (CH); KLABUNDE, Ralf, 2400 Le Locle (CH); LOCHON, Thomas, 2400 Le Locle (CH)
(74) Representative: Orr, Robert
(86) International application number: PCT/EP2012/070253
(87) International publication number: WO 2013/053885

(56) References cited:
- DE-U1- 29 903 342
- US-A1- 2006 083 603
- US-A1- 2007 260 246

## Description

### Background

Bone anchors may be used in orthopedic surgery to fix bone during the healing or fusion process. In spinal surgery, bone anchors may be used with spinal fixation elements, such as spinal rods, to stabilize multiple vertebrae either rigidly, in which no relative motion between the vertebrae is desired, and dynamically, in which limited, controlled motion between the vertebrae is desired. A closure mechanism is typically used to secure the spinal fixation element to the bone anchor. While various types of closure mechanisms have been developed, the most commonly used closure mechanisms engage the bone anchor through a threaded connection. Conventional threaded connections attempt to minimize the lateral transfer of the axial tightening force in an effort to minimize deformation of the bone anchor and splaying of the bone anchor. Such threaded connections can be difficult and expensive to manufacture. Accordingly, there is a need for improved threaded connections between the closure mechanism and the bone anchor.

US Patent Publication No. US-2007/0260246 discloses a locking device for securing a rod in a holding element connected to a shank of a bone screw. The holding element has a recess having a U-shaped cross-section for receiving the rod, with two legs which are open at one end and an inner thread on the open legs. The locking device takes the form of a locking element, and has an outer thread in at least one section of its outer wall, the outer thread cooperating with the inner thread of the legs.

German Utility Model Publication No.DE-29903342 discloses a vertebral implant comprising a screw and a holding element. The screw comprises a fork-shaped element which receives the holding element, and an inner wall of the fork-shaped element has a threaded section in which a screw can be inserted, for applying pressure to the holding element.

US Patent Publication No. US-2006/0083603 discloses an open-headed bone screw including a reverse angled threadform with anti-splay clearance. The screw includes inner and outer helical threads, each with respective leading stab flanks and trailing load flanks, the load flanks mutually engaging when an inner member with the inner threads is advanced into an outer member with the outer threads. The inner load flanks form acute angles with a helical axis, while the outer load flanks form obtuse angles with the helical axis. The clearance is formed between the stab flanks of the inner and outer threads.

### Summary

Disclosed herein are improved bone anchor assemblies and, in particular, improved bone anchor assemblies used in connection with spinal fixation elements to fix multiple vertebrae.

The present invention provides a bone anchor assembly as claimed in claim 1.

The bone anchor assembly includes a bone anchor having a proximal head and a distal shaft configured to engage bone, a receiver member for receiving a spinal fixation element to be coupled to the bone anchor, and a closure mechanism. The receiver member has a proximal end having a pair of spaced apart arms defining a recess therebetween and a distal end having a distal end surface defining an opening through which at least a portion of the bone anchor extends. The closure mechanism is positionable between the arms to capture a spinal fixation element within the receiver member and fix the spinal fixation element with respect to the receiver member. The closure mechanism includes an outer thread for engaging an inner thread on the arms of the receiver member. The inner thread and the outer thread have a thread angle between 5° and 25°.

Further features of the bone anchor assembly are defined in the dependent claims.

### Brief Description of the Figures

These and other features and advantages of the devices and methods disclosed herein will be more fully understood by reference to the following detailed description in conjunction with the attached drawings in which like reference numerals refer to like elements through the different views. The drawings illustrate principles of the devices and methods disclosed herein and, although not to scale, show relative dimensions.
FIGURE 1 is a perspective view of an exemplary embodiment of a bone anchor assembly;
FIGURE 2 is an exploded view of the bone anchor assembly of FIGURE 1;
FIGURE 3 is a top view of the bone anchor of the bone anchor assembly FIGURE 1;
FIGURE 4 is a side view in cross section of the bone anchor assembly of FIGURE 1;
FIGURE 5 is a cross sectional view of the inner thread of receiver member and the outer thread of the closure mechanism of the bone anchor assembly FIGURE 1;
FIGURE 6 is a perspective view of an alternative embodiment of a closure mechanism;
FIGURE 7 is a top view of the closure mechanism of FIGURE 6;
FIGURE 8 is a side view of the closure mechanism of FIGURE 6;
FIGURE 9 is a side view in cross section of the closure mechanism of FIGURE 6;
FIGURE 10 is a perspective view of the closure mechanism of FIGURE 6, illustrating an exemplary embodiment of an instrument for driving the closure mechanism;
FIGURES 11A-11B are side views (FIGURE 11B in cross section) of an exemplary instrument for engaging and driving a closure mechanism, illustrating the instrument prior to connection to a closure mechanism;
FIGURES 12A-12B are side views (FIGURE 12B in cross section) of the exemplary instrument of FIGURES 11A-11B, illustrating the initial engagement of the instrument to a closure mechanism; and
FIGURES 13A-13B are side views (FIGURE 13B in cross section) of the exemplary instrument of FIGURES 11A-11B, illustrating the outer sleeve of the instrument advanced into engagement with the closure mechanism.

### Detail Description of Exemplary Embodiments

Certain exemplary embodiments will now be described to provide an overall understanding of the principles of the structure, function, manufacture, and use of the devices disclosed herein. One or more examples of these embodiments are illustrated in the accompanying drawings. Those of ordinary skill in the art will understand that the devices specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

The articles "a" and "an" are used herein to refer to one or to more than one (i.e. to at least one) of the grammatical object of the article. By way of example, "an element" means one element or more than one element.

The terms "comprise," "include," and "have," and the derivatives thereof, are used herein interchangeably as comprehensive, open-ended terms. For example, use of "comprising," "including," or "having" means that whatever element is comprised, had, or included, is not the only element encompassed by the subject of the clause that contains the verb.

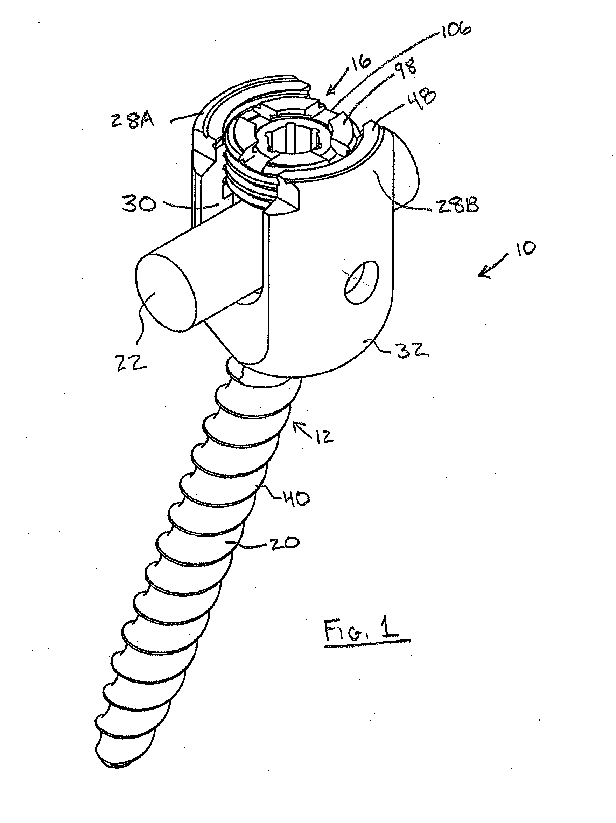

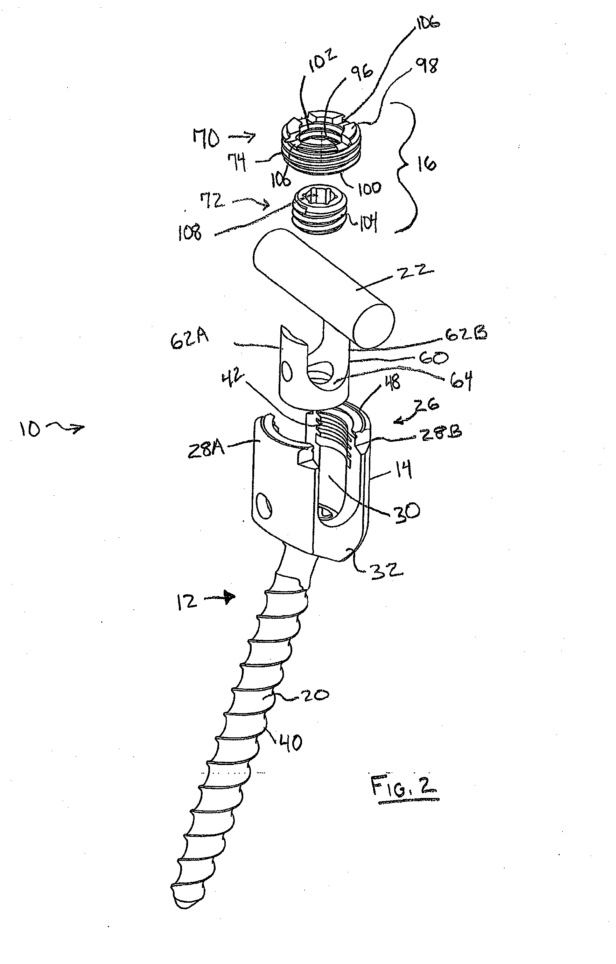

FIGURES 1-5 illustrate an exemplary embodiment of a bone anchor assembly 10 including a bone anchor 12, a receiver member 14 for receiving a spinal fixation element, such as a spinal rod 22, to be coupled to the bone anchor 12, and a closure mechanism 16 to capture a spinal fixation element within the receiver member 14 and fix the spinal fixation element with respect to the receiver member 14. The bone anchor 12 includes a proximal head 18 and a distal shaft 20 configured to engage bone. The receiver member 14 has a proximal end 26 having a pair of spaced apart arms 28A, 28B defining a recess 30 therebetween and a distal end 32 having a distal end surface 34 defining opening through which at least a portion of the bone anchor 12 extends. The closure mechanism 16 may be positionable between and may engage the arms 28A, 28B to capture a spinal fixation element, e.g., spinal rod 22, within the receiver member 14 and fix the spinal fixation element with respect to the receiver member 14.

Continuing to refer to FIGURES 1-5, the proximal head 16 of the bone anchor 12 in the exemplary embodiment is generally in the shape of a truncated sphere having a planar proximal surface 36 and an approximately spherically shaped distal surface 38. The exemplary bone anchor assembly is a polyaxial bone screw designed for posterior implantation in the pedicle or lateral mass of a vertebra. In this regards, the proximal head 18 of the bone anchor 12 engages the distal end 32 of the receiver member 14 in a ball and socket like arrangement in which the proximal head 18, and thus the distal shaft 20, can pivot relative to the receiver member 14. The distal surface 38 of the proximal head 18 of the bone anchor 12 and the mating surface of the within the distal end 32 of the receiver member 14 may have any shape that facilitates this ball and socket like arrangement, including, for example, spherical (as illustrated), toroidal, conical, frustoconical, and any combinations of these shapes.

The distal shaft 20 of the bone anchor 12 may be configured to engage bone and, in the illustrated embodiment, includes an external bone engaging thread 40. The thread form for the distal shaft 20, including the number of threads, the pitch, major and minor diameter, and thread shape, may be selected to facilitate connection with bone. Examples of exemplary thread forms are disclosed in U.S. Patent Application Serial No. 13/110,378, filed May 18, 2011 (published as US-2011/0288599), and U.S. Provisional Patent Application Serial No. 61/527,389, filed August 25, 2011 (see WO-2013/028851)-. Alternatively, the distal shaft 20 may include other structures for engaging bone, including a hook. The distal shaft 20 of the bone anchor 12 may be cannulated, having a central passage or cannula extending the length of the bone anchor to facilitate delivery of the bone anchor over a guide wire in, for example, minimally invasive procedures. The other components of the bone anchor assembly, including the closure member 16, the receiver member 14, and the compression member 60 (discussed below) may be cannulated or otherwise have an opening to permit the respective component to be delivered over a guide wire. The distal shaft 20 may also include one or more side wall openings or fenestrations that communicate with the cannula to permit bone in-growth or to permit the dispensing of bone cement or other materials through the bone anchor 10. The side wall openings may extend radially from the cannula through the side wall of the distal shaft 20. Exemplary systems for delivering bone cement to the bone anchor assembly 10 and alternative bone anchor configurations for facilitating cement delivery are described in U.S. Patent Application Publication No. 2010/0114174. The distal shaft 20 of the bone anchor 12 may also be coated with materials to permit bone growth, such as, for example, hydroxyl apatite, and the bone anchor assembly 10 may be coated all or in-part with anti-infective materials, such as, for example, tryclosan.

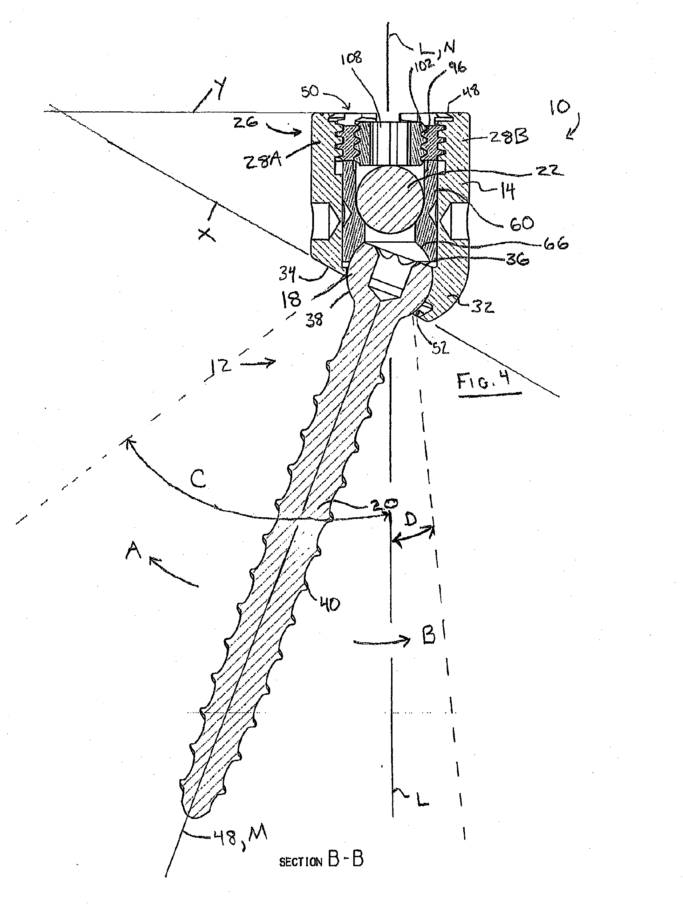

Continuing to refer to FIGURES 1-5, the proximal end 26 of the receiver member 14 of the exemplary bone anchor assembly 10 includes a pair of spaced apart arms 28A, 28B defining the U-shaped recess 30 therebetween for receiving a spinal fixation element. e.g., spinal rod 22. Each arm 28A, 28B of the proximal end 26 of the receiver member 14 extends from the distal end 32 of the receiver member 14 to a free end. The outer surface of each arm 28A, 28B may include a feature, such as a recess, dimple, notch, projection, or the like, to facilitate connection of the receiver member 14 and, thus, the bone anchor assembly 10, to instruments. For example, the outer surface of each arm 28A, 28B may include an arcuate groove at the respective free end of the arms. Such grooves are described in more detail in U.S. Patent No. 7,179,261. At least a portion of the proximal end surface 48 of the receiver member 12 defines a plane Y, as illustrated in FIGURE 4. The receiver member 14 has a central longitudinal axis L.

The distal end 32 of the receiver member 14 includes a distal end surface 34 which is generally annular in shape defining a circular opening through which at least a portion of the bone anchor 12 extends. For example, the distal shaft 20 of the bone anchor 12 may extend through the opening. At least a portion of the distal end surface 34 defines a plane X.

The exemplary bone anchor assembly is a rigid polyaxial screw in which the bone anchor 12 can be selectively fixed relative to the receiver member 14. Prior to fixation, the bone anchor 12 is movable relative to the receiver member 14 within a cone of angulation generally defined by the geometry of the distal end 32 of the receiver member and the proximal head 18 of the bone anchor 12. The exemplary bone anchor is a favored-angle polyaxial screw in which the cone of angulation is biased in one direction. In this manner, the bone anchor 12 is movable relative to the receiver member 14 in at least a first direction, indicated by arrow A in FIGURE 4, at a first angle C relative to the central longitudinal axis L of the receiver member 14. The bone anchor 12 is also movable in at least a second direction, indicated by arrow B in FIGURE 4, at a second angle D relative to the longitudinal axis L. The first angle C is greater than the second angle D and, thus, the shaft 20 of the bone anchor 12 is movable more in the direction indicated by arrow A than in the direction indicated by arrow B. The distal shaft 20 of the bone anchor 12 defines a neutral axis 48 with respect to the receiver member 14. In the exemplary favored-angle polyaxial screw embodiment, the neutral axis 48 is oriented perpendicular to the plane X defined by the distal end surface 34 and intersects the center point of the opening in the distal end surface 34 through which the distal shaft 20 of the bone anchor 12 extends. The neutral axis 48 is oriented at an angle to the central longitudinal axis L of the receiver member 14 in one exemplary manner of providing biased angulation of the bone anchor 12. In one exemplary manner of providing biased angulation, the plane Y defined by at least a portion of the proximal end surface 48 of the receiver member 14 intersects the plane X defined by at least a portion of the distal end surface 34 of the receiver member 12. In addition (or in the alternative), the proximal end 26 of the receiver member 14 may include a proximal first bore 50 coaxial with a first central longitudinal axis N (which is coincident with longitudinal axis L) and a distal second bore 52 coaxial with a second central longitudinal axis M (which is coincident with neutral axis 48) and the first central longitudinal axis N and second central longitudinal axis M can intersect one another. The angle between the plane X and plane Y and the angle between the axis L and axis M can be selected to provided the desired degree of biased angulation. Examples of favored angled polyaxial screws are described in more detail in U.S. Patent Application Publication 2003/0055426 and U.S. Patent No. 6,736,820. In alternative embodiments, the bone anchor assembly can be a conventional (non-biased) polyaxial screw in which the bone anchor pivots in the same amount in every direction and has a neutral axis that is coincident with the central longitudinal axis L of the receiver member.

The spinal fixation element, e.g., spinal rod 22, may either directly contact the proximal head 18 of the bone anchor 12 or may contact an intermediate element, e.g., a compression member 60, positioned within the receiver member 14 and interposed between the spinal rod 22 and the proximal head 18 of the bone anchor 12 to compress the distal outer surface 38 of the proximal head 18 into direct, fixed engagement with the distal inner surface of the receiver member 18. In the exemplary embodiment, the compression member 60 includes a pair of spaced apart arms 62A and 62B defining a U-shaped seat 64 for receiving the spinal rod 22 and a distal surface 66 for engaging the proximal head 18 of the bone anchor 12.

The proximal end 26 of the receiving member 14 may be configured to receive a closure mechanism 16 positionable between and engaging the arms 28A and 28B of the receiver member to capture a spinal fixation element, e.g., a spinal rod 22, within the receiver member 14, to fix the spinal rod 22 relative to the receiver member 14, and to fix the bone anchor 12 relative to the receiver member 14. In certain exemplary embodiments, the closure mechanism 16 may be a single set screw having an outer thread for engaging an inner thread 42 provided on the arms 28A and 28B of the receiver member 14. In the exemplary embodiment, the closure mechanism 16 comprises an outer set screw 70 positionable between and engaging the arms 28A and 28B of the receiver member 14 and an inner set screw 72 positionable within the outer set screw 70. The outer set screw 70 is operable to act on the compression member 60 to fix the bone anchor 12 relative to the receiver member 14. The inner set screw 72 is operable to act on the spinal rod 22 to fix the spinal rod 22 relative to the receiver member 14. In this manner, the closure mechanism 16 permits the bone anchor 12 to be fixed relative to the receiver member 14 independently of the spinal rod 22 being fixed to the receiver member 14. In particular, the outer set screw 70 can engage the proximal end surfaces of the arms 62A and 62B of the compression member 60 to force the distal surface 66 of the compression member 60 into contact with the proximal head 18 of bone anchor 12, which in turn forces the distal surface 38 of the proximal head 18 into the fixed engagement with the distal inner surface of the receiver member 14. The inner set screw 72 can engage the spinal rod 22 to force the spinal rod 22 into fixed engagement with the rod seat 64 of the compression member 60.

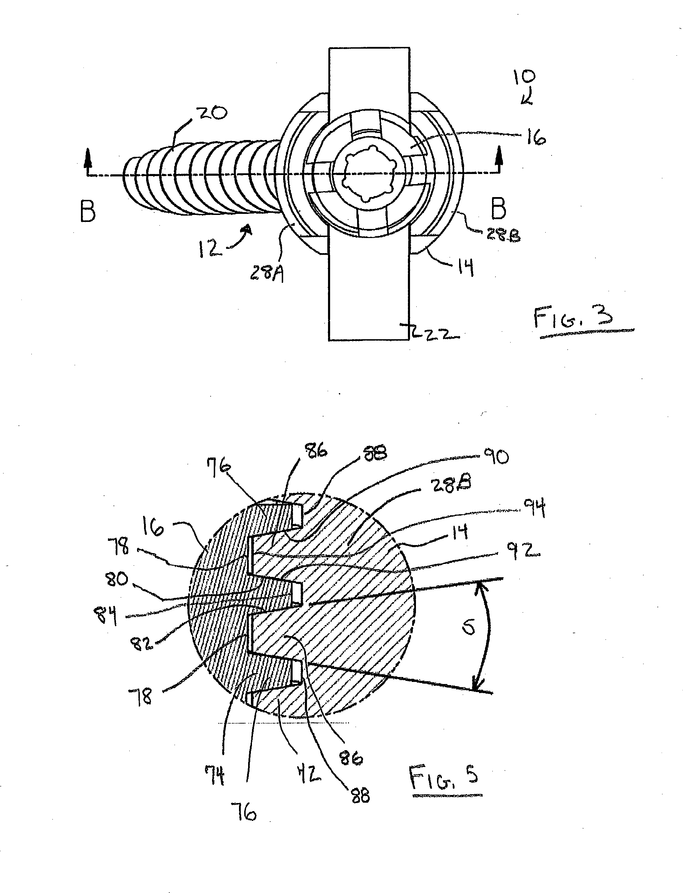

The outer set screw 70 of the exemplary closure mechanism 16 includes a first outer thread 74 for engaging the complementary inner thread 42 on the arms 28A and 28B of the receiver member 14. As illustrated in FIGURE 5, the inner thread 42 and the outer thread 74 have a thread angle S between 5° and 25°. In certain embodiments, the thread angle can be between 5° and 20°. In one preferred embodiment (illustrated in FIGURE 5) the thread angle is approximately 10° and another preferred embodiment the thread angle is approximately 15°. A thread with a thread angle within these ranges minimizes the lateral transfer of the axial tightening force compared to conventional threads such as an acme thread or a metric thread and, thus, minimizes instances of splaying of the arms 28A and 28B of the receiver member 14 during tightening of the outer set screw 74. In addition, a thread with such a thread angle is less difficult and less expensive to manufacture compared to a square thread.

The outer thread 74 comprises a plurality of uniform thread ridges 76 separated from one another by a root 78. Each thread ridge includes a first flank 80 extending from a root 78, a second flank 82 extending from a root 78, a crest 84 connecting the first flank 80 and second flank 82. A root 78 and the crest 84 of an adjacent thread ridge 76 may be planar and may be oriented parallel to a central axis of the outer set screw 70. The first flank 80 and the second flank 82 may also be planar. The inner thread 42 comprises a plurality of uniform thread ridges 86 separated from one another by a root 88. Each thread ridge includes a first flank 90 extending from a root 88, a second flank 92 extending from a root 88, a crest 94 connecting the first flank 90 and second flank 92. A root 88 and the crest 94 of an adjacent thread ridge 86 may be planar and may be oriented parallel to the longitudinal axis L of the receiver member 14. The first flank 90 and the second flank 92 may also be planar. For both the inner thread 42 and the outer thread 74, the number of threads, the pitch, and the major and minor diameter may be selected to facilitate the connection between the outer set screw 70 and the receiver member 14 and transfer of the desired axial tightening force.

The outer set screw 74 may have a central passage 96 from a top surface 98 of the outer set screw 74 to a bottom surface 100 of the outer set screw 74 for receiving the inner set screw 72. The central passage 96 may have an inner thread 102 for engaging a complementary outer thread 104 on the inner set screw 72. The thread form for the inner thread 102 and the outer thread 104, including the number of threads, the pitch, major and minor diameter, and thread shape, may be selected to facilitate connection between the components and transfer of the desired axial tightening force. In the illustrated embodiment, for example, the inner thread 102 and the outer thread 104 are M7 x 1 metric threads. The top surface 98 of the outer set screw 74 may have one or more drive features to facilitate rotation and advancement of the outer set screw 74 relative to the receiver member 14. In the exemplary embodiment, the drive features are a plurality of cut-outs 106 spaced-apart about the perimeter of the top surface 98. In the inner set screw 104 may include drive feature for receiving an instrument to rotate and advance the inner set screw 72 relative to the outer set screw 74. In the illustrated embodiment, for example, the inner set screw 104 includes a central passage 108 having a plurality of spaced apart, longitudinally oriented cut-outs for engaging complementary features on an instrument.

The exemplary bone anchor assembly 10 may be used with a spinal fixation element such as rigid spinal rod 22. The spinal rod 22 may be constructed from titanium, titanium alloys, stainless steel, cobalt chrome, PEEK, or other materials suitable for rigid fixation. Alternatively, the spinal fixation element may be a dynamic stabilization member that allows controlled mobility between the instrumented vertebrae.

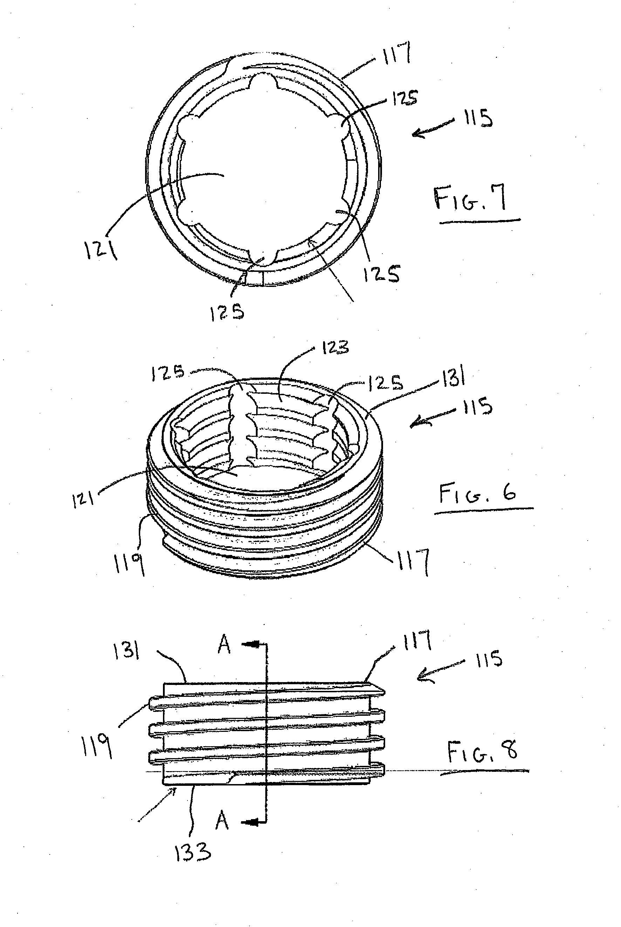

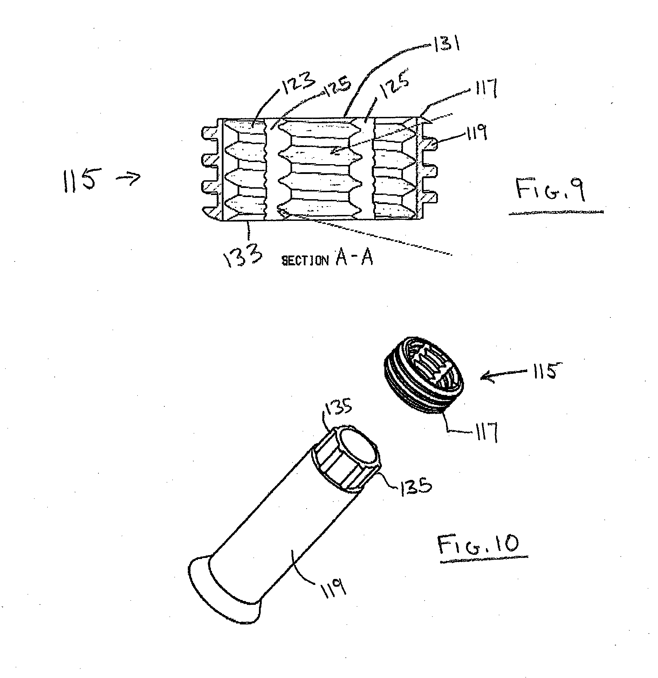

FIGURES 6-10 illustrate an alternative embodiment of a closure mechanism 115 for a bone anchor assembly. The closure mechanism 115 includes an outer set screw 117 having an outer thread 119 for engaging an inner thread on a receiver member of a polyaxial screw. The outer thread 119 can be similar to the outer thread 74 of the outer set screw 70 described above or may be a square thread (as illustrated) or other thread suitable for capturing and fixing a spinal rod to a receiver member of a polyaxial screw. The outer set screw 117 may include a central passage 121 having an inner thread 123. The central passage 121 includes a plurality of spaced apart notches 125 oriented parallel to the central longitudinal axis of the central passage 121. The notches 125 are non-threaded and interrupt the inner thread 123. The notches 125 open at and extend from the top surface 131 of the outer set screw 117 to facilitate placement of an instrument, such as exemplary instrument 119 having drive features 135 complementary in size and shape to the notches 125, from the top of the outer set screw 117 (i.e., in a proximal to distal direction). The notches 125 may extend to the bottom surface 133 of the outer set screw 117, as in the illustrated embodiment, or may terminate prior to the bottom surface 133 a distance from the top surface 131 sufficient to permit proper engagement with the instrument. Providing the outer set screw 117 with an internal instrument drive feature reduces the height of the outer set screw 117, and thus reduces the profile (i.e., the vertical height) of the receiver member 14, compared to the closure mechanism 16 described above.

The closure mechanism 115 may include an inner set screw, such as inner set screw 72 described above, for positioning within the central passage 121.

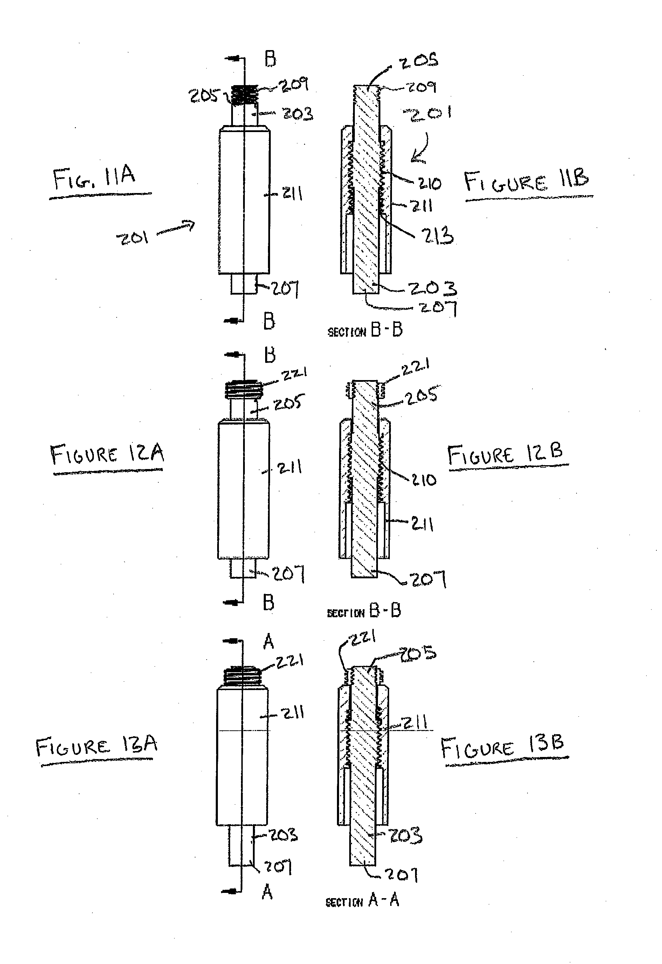

FIGURES 11A-13B illustrate an exemplary instrument 201 for engaging and driving an outer set screw of a closure mechanism for a bone anchor assembly, such as, for example, the closure mechanism 16 and the closure mechanism 115. The instrument 201 includes a tubular inner shaft 203 having a proximal end 205 and a distal end 207. The proximal end 205 includes a first outer thread 209 for engagement with an inner thread on an outer set screw. The inner shaft 203 includes a second outer thread 210 between the proximal end 205 and the distal end 207. The instrument 201 also includes an outer sleeve 211 positionable about the inner shaft 203. The outer sleeve 211 is generally cylindrical in shape and includes an inner thread 213 for engagement with the second outer thread 209. Rotation of the outer sleeve 211 relative to the inner shaft 203 results in axial motion of the outer sleeve 211 relative to the inner sleeve 203.

In use, an outer set screw 221 with an internally threaded central passage is connected to the proximal end 205 of the inner shaft 203 by rotational, threaded engagement of the first outer thread 209 with the internally thread central passage of the outer set screw 221, as illustrated in FIGURES 12A and 12B. The proximal end of the outer sleeve 211 is advanced into contact with the top surface of the outer set screw 221 by rotating the outer sleeve 211 relative to the inner shaft 203, as illustrated in FIGURES 13A and 13B. In this manner, the outer set screw 221 is fixed to the instrument 201. Continued rotation of the outer sleeve 211 causes the outer set screw 221 to rotate thereby permitting the outer set screw 221 to be threaded into the receiver member of a bone anchor assembly. The instrument 201 uses the internally threaded central passage of the outer set screw to engage and drive to the outer set screw 221 without the need for a separate drive feature on the outer set screw 221.

While the devices of the present invention have been particularly shown and described with reference to the exemplary embodiments thereof, those of ordinary skill in the art will understand that various changes may be made in the form and details herein. Those of ordinary skill in the art will recognize or be able to ascertain many equivalents to the exemplary embodiments described specifically herein by using no more than routine experimentation. Such equivalents are intended to be encompassed by the scope of the present invention and the appended claims.

Another bone anchor assembly is disclosed which comprises:
a bone anchor having a proximal head and a distal shaft configured to engage bone,
a receiver member for receiving a spinal fixation element to be coupled to the bone anchor, the receiver member having
a proximal end having a pair of spaced apart arms defining a recess therebetween for receiving a spinal rod, the arms including a first inner thread,
a distal end having a distal end surface defining opening through which at least a portion of the bone anchor extends, the bone anchor being movable relative to the receiver member,
a compression member positioned within the receiver member, the compression member including a seat for receiving the rod and a distal surface for engaging the proximal head of the bone anchor,
an outer set screw positionable between and engaging the arms, the outer set screw including a first outer thread for engaging the first inner thread, the outer set screw operable to act on the compression member to fix the bone anchor relative to the receiver member, the first inner thread and the first outer thread having a thread angle between 5° and 25°, the outer set screw having a central passage from a top surface of the outer set screw to a bottom surface of the outer set screw, the central passage having a second internal thread, and
an inner set screw positionable within the central passage, the inner set screw having a second outer thread for engaging the second inner thread, the inner set screw operable to act on the spinal rod to fix the spinal rod relative to the receiver member.

The first inner thread and the first outer thread may each comprise a plurality of uniform thread ridges, the thread ridges being separated from one another by a root, each thread ridge including a first flank extending from a root, a second flank extending from a root, and a crest connecting the first flank and second flank. A root and the crest of an adjacent thread ridge may be planar and oriented parallel to a central axis of the closure mechanism. The first flank and the second flank may be planar. The thread angle may be between 5° and 20°. The thread angle may be approximately 15°. The thread angle may be approximately 10°. The central passage of the outer set screw may include a plurality of spaced apart notches oriented parallel to a central axis of the central passage. The notches may extend from the top surface of the closure mechanism. The notches may extend from the top surface of the closure mechanism to the bottom surface of the closure mechanism. The bone anchor may be movable relative to the receiver member in at least a first direction at a first angle relative to a central axis of the receiver member and in at least a second direction at a second angle relative to the central axis of the receiver member, the second angle being greater than the first angle. The receiver member may define a proximal first bore coaxial with a first longitudinal axis and a distal second bore coaxial with a second longitudinal axis, the first and second longitudinal axes intersecting one another, and the first and second bores communicating with one another. The proximal end of the receiver member may include a proximal end surface that defines a first plane and wherein the distal end surface may define a second plane, the first plane and second plan intersecting one another.

Another bone anchor assembly is disclosed which comprises:
a bone anchor having a proximal head and a distal shaft configured to engage bone,
a receiver member for receiving a spinal fixation element to be coupled to the bone anchor, the receiver member having
a proximal end having a proximal end surface and a pair of spaced apart arms defining a recess therebetween for receiving a spinal rod, the arms including a first inner thread, the proximal end surface defining a first plane a distal end having a distal end surface defining an opening through which at least a portion of the bone anchor extends, the bone anchor being movable relative to the receiver member, the distal end surface defining a second plane, the first plane and the second plane intersecting one another,
a compression member positioned within the receiver member, the compression member including pair of spaced apart arms defining a u-shaped seat for receiving the rod and a distal surface for engaging the proximal head of the bone anchor,
an outer set screw positionable between and engaging the arms, the outer set screw including a first outer thread for engaging the first inner thread, the outer set screw operable to act on the compression member to fix the bone anchor relative to the receiver member, the first inner thread and the first outer thread having a thread angle between 5° and 25°, the outer set screw having a central passage from a top surface of the outer set screw to a bottom surface of the outer set screw, the central passage having a second internal thread, and
an inner set screw positionable within the central passage, the inner set screw having a second outer thread for engaging the second inner thread, the inner set screw operable to act on the spinal rod to fix the spinal rod relative to the receiver member.

## Claims

1. A bone anchor assembly (10) comprising:
a bone anchor (12) having a proximal head (18) and a distal shaft (20) configured to engage bone,
a receiver member (14) for receiving a spinal fixation element (22) to be coupled to the bone anchor, the receiver member having
a proximal end (26) having a pair of spaced apart arms (28A, 28B) defining a recess (30) therebetween, the arms including an inner thread (42),
a distal end (32) having a distal end surface (34) defining an opening through which at least a portion of the bone anchor extends, and
a closure mechanism (16; 115) positionable between the arms to capture the spinal fixation element within the receiver member and fix the spinal fixation element with respect to the receiver member, the closure mechanism including an outer thread (74) for engaging the inner thread on the arms of the receiver member,
**characterised in that** the inner thread and the outer thread have a thread angle between 5° and 25°.

2. The bone anchor assembly of claim 1, wherein the inner thread and the outer thread each comprise a plurality of uniform thread ridges (86; 76), the thread ridges being separated from one another by a root (88; 78), each thread ridge including a first flank (90; 80) extending from a root, a second flank (92; 82) extending from a root, and a crest (94; 84) connecting the first flank and second flank.

3. The bone anchor assembly of claim 2, wherein a root and the crest of an adjacent thread ridge are planar and are oriented parallel to a central axis of the closure mechanism.

4. The bone anchor assembly of claim 3, wherein the first flank and the second flank are planar.

5. The bone anchor assembly of claim 1, wherein the thread angle is between 5° and 20°.

6. The bone anchor assembly of claim 1, wherein the thread angle is approximately 15°.

7. The bone anchor assembly of claim 1, wherein the thread angle is approximately 10°.

8. The bone anchor assembly of claim 1, wherein the closure mechanism includes a central passage (96; 121) from a top surface (98; 131) of the closure mechanism to a bottom surface (100; 133) of the closure mechanism.

9. The bone anchor assembly of claim 8, wherein the central passage includes an inner thread (102; 123).

10. The bone anchor assembly of claim 8, wherein the central passage includes a plurality of spaced apart notches (125) oriented parallel to a central axis of the central passage.

11. The bone anchor assembly of claim 10, wherein the notches extend from the top surface of the closure mechanism.

12. The bone anchor assembly of claim 11, wherein the notches extend from the top surface of the closure mechanism to the bottom surface of the closure mechanism.

13. The bone anchor assembly of claim 9, wherein the closure mechanism further includes an inner set screw (72) for positioning within the central passage, the inner set screw having an outer thread (104) for engaging the inner thread of the central passage.

14. The bone anchor assembly of claim 1, wherein the bone anchor is movable relative to the receiver member.

15. The bone anchor assembly of claim 14, wherein the bone anchor is movable relative to the receiver member in at least a first direction (A) at a first angle (C) relative to a central axis (L) of the receiver member and in at least a second direction (B) at a second angle (D) relative to the central axis of the receiver member, the second angle being greater than the first angle.

## Patentansprüche

1. Knochenankeranordnung (10), umfassend:
einen Knochenanker (12) mit einem proximalen Kopf (18) und einem distalen Schaft (20), ausgelegt in Knochen einzugreifen,
ein Aufnahmeglied (14) zum Aufnehmen eines an den Knochenanker gekoppelten Wirbelsäulenfixierungselements (22), wobei das Aufnahmeglied aufweist
ein proximales Ende (26) mit einem Paar zueinander beabstandeter Arme (28A, 28B), die eine dazwischenliegende Vertiefung (30) definieren, wobei die Arme ein Innengewinde (42) einschließen,
ein distales Ende (32) mit einer distalen Endoberfläche (34), die eine Öffnung definiert, durch die sich zumindest ein Teil des Knochenankers erstreckt, und
einen zwischen den Armen positionierbaren Schließmechanismus (16; 115) zum Ergreifen des Wirbelsäulenfixierungselements in dem Aufnahmeglied und Fixieren des Wirbelsäulenfixierungselements in Bezug auf das Aufnahmeglied, wobei der Schließmechanismus ein Außengewinde (74) zum Eingriff in das Innengewinde an den Armen des Aufnahmeglieds einschließt,
**dadurch gekennzeichnet, dass** das Innengewinde und das Außengewinde einen Flankenwinkel zwischen 5° und 25° aufweisen.

2. Knochenankeranordnung nach Anspruch 1, wobei das Innengewinde und das Außengewinde jeweils eine Mehrzahl einheitlicher Gewindezähne (86; 76) umfassen, wobei die Gewindezähne voneinander durch einen Gewindegrund (88; 78) getrennt sind, wobei jeder Gewindezahn eine sich von einem Gewindegrund erstreckende erste Flanke (90; 80), eine sich von einem Gewindegrund erstreckende zweite Flanke (92; 82) und eine die erste Flanke und die zweite Flanke verbindende Gewindespitze (94; 84) aufweist.

3. Knochenankeranordnung nach Anspruch 2, wobei ein Gewindegrund und die Gewindespitze eines benachbarten Gewindezahns eben und parallel zu einer Mittelachse des Schließmechanismus ausgerichtet sind.

4. Knochenankeranordnung nach Anspruch 3, wobei die erste Flanke und die zweite Flanke eben sind.

5. Knochenankeranordnung nach Anspruch 1, wobei der Flankenwinkel zwischen 5° und 20° liegt.

6. Knochenankeranordnung nach Anspruch 1, wobei der Flankenwinkel etwa 15° beträgt.

7. Knochenankeranordnung nach Anspruch 1, wobei der Flankenwinkel etwa 10° beträgt.

8. Knochenankeranordnung nach Anspruch 1, wobei der Schließmechanismus eine mittige Durchführung (96; 121) von einer Oberseite (98; 131) des Schließmechanismus zu einer Unterseite (100; 133) des Schließmechanismus einschließt.

9. Knocheankeranordnung nach Anspruch 8, wobei die mittige Durchführung ein Innengewinde (102; 123) einschließt.

10. Knochenankeranordnung nach Anspruch 8, wobei die mittige Durchführung eine Mehrzahl zueinander beabstandeter Kerben (125) einschließt, die parallel zu einer Mittelachse der mittigen Durchführung ausgerichtet sind.

11. Knochenankeranordnung nach Anspruch 10, wobei die Kerben sich von der Oberseite des Schließmechanismus erstrecken.

12. Knochenankeranordnung nach Anspruch 11, wobei die Kerben sich von der Oberseite des Schließmechanismus zu der Unterseite des Schließmechanismus erstrecken.

13. Knochenankeranordnung nach Anspruch 9, wobei der Schließmechanismus weiterhin einen inneren Gewindestift (72) zum Positionieren in der mittigen Durchführung einschließt, wobei der innere Gewindestift ein Außengewinde (104) zum Eingriff in das Innengewindes der mittigen Durchführung aufweist.

14. Knochenankeranordnung nach Anspruch 1, wobei der Knochenanker relativ zu dem Aufnahmeglied bewegbar ist.

15. Knochenankeranordnung nach Anspruch 14, wobei der Knochenanker relativ zu dem Aufnahmeglied in zumindest einer ersten Richtung (A) unter einem ersten Winkel (C) relativ zu einer Mittelachse (L) des Aufnahmeglieds und in zumindest einer zweiten Richtung (B) unter einem zweiten Winkel (D) relativ zu der Mittelachse des Aufnahmeglieds bewegbar ist, wobei der zweite Winkel größer als der erste Winkel ist.

## Revendications

1. Ensemble d'ancrage d'os (10) comprenant :
un ancrage d'os (12) ayant une tête proximale (18) et un arbre distal (20) configuré pour venir en prise avec l'os,
un élément de réception (14) pour recevoir un élément de fixation de rachis (22) à coupler à l'ancrage d'os, l'élément de réception ayant
une extrémité proximale (26) ayant une paire de bras espacés (28A, 28B) définissant un évidement (30) entre eux, les bras incluant un filetage intérieur (42),
une extrémité distale (32) ayant une surface d'extrémité distale (34) définissant une ouverture à travers laquelle s'étend au moins une portion de l'ancrage d'os, et
un mécanisme de fermeture (16 ; 115) apte à être positionné entre les bras pour capturer l'élément de fixation du rachis dans l'élément de réception et pour fixer l'élément de fixation spinal relativement à l'élément de réception, le mécanisme de fermeture incluant un filetage extérieur (74) pour venir en prise avec le filetage intérieur sur les bras de l'élément de réception,
**caractérisé en ce que** le filetage intérieur et le filetage extérieur ont un angle de filetage entre 5° et 25°.

2. Ensemble d'ancrage d'os selon la revendication 1, dans lequel le filetage intérieur et le filetage extérieur comprennent chacun une pluralité de crêtes de filetage uniformes (86 ; 76), les crêtes de filetage étant séparées les unes des autres par une racine (88 ; 78), chaque crête de filetage incluant un premier flanc (90 ; 80) s'étendant d'une racine, un deuxième flanc (92, 82) s'étendant d'une racine et une arête (94 ; 84) reliant le premier flanc et le deuxième flanc.

3. Ensemble d'ancrage d'os selon la revendication 2, dans lequel une racine et l'arête d'une crête de filetage adjacente sont planes et sont orientées parallèlement à un axe central du mécanisme de fermeture.

4. Ensemble d'ancrage d'os selon la revendication 3, dans lequel le premier flanc et le deuxième flanc sont plans.

5. Ensemble d'ancrage d'os selon la revendication 1, dans lequel l'angle de filetage est entre 5° et 20°.

6. Ensemble d'ancrage d'os selon la revendication 1, dans lequel l'angle de filetage est approximativement de 15°.

7. Ensemble d'ancrage d'os selon la revendication 1, dans lequel l'angle de filetage est approximativement de 10°.

8. Ensemble d'ancrage d'os selon la revendication 1, dans lequel le mécanisme de fermeture comprend un passage central (96 ; 121) d'une surface supérieure (98 ; 131) du mécanisme de fermeture à une surface inférieure (100 ; 133) du mécanisme de fermeture.

9. Ensemble d'ancrage d'os selon la revendication 8, dans lequel le passage central comprend un filetage intérieur (102 ; 123).

10. Ensemble d'ancrage d'os selon la revendication 8, dans lequel le passage central inclut une pluralité d'encoches espacées (125) orientées parallèlement à un axe central du passage central.

11. Ensemble d'ancrage d'os selon la revendication 10, dans lequel les encoches s'étendent de la surface supérieure du mécanisme de fermeture.

12. Ensemble d'ancrage d'os selon la revendication 11, dans lequel les encoches s'étendent de la surface supérieure du mécanisme de fermeture à la surface inférieure du mécanisme de fermeture.

13. Ensemble d'ancrage d'os selon la revendication 9, dans lequel le mécanisme de fermeture comprend en outre une vis de réglage intérieure (72) pour le positionnement dans le passage central, la vis de réglage intérieure ayant un filetage extérieur (104) destiné à venir en prise avec le filetage intérieur du passage central.

14. Ensemble d'ancrage d'os selon la revendication 1, dans lequel l'ancrage d'os est déplaçable relativement à l'élément de réception.

15. Ensemble d'ancrage d'os selon la revendication 14, dans lequel l'ancrage d'os est déplaçable relativement à l'élément de réception dans au moins une première direction (A) à un premier angle (C) relativement à un axe central (L) de l'élément de réception et dans au moins une seconde direction (B) à un second angle (D) relativement à l'axe central de l'élément de réception, le second angle étant plus grand que le premier angle.
